# EUROPÄISCHE PATENTANMELDUNG

(11) **EP 2 263 750 A1**
(43) Veröffentlichungstag der Anmeldung: **22.12.2010**
(21) Anmeldenummer: 10163994.6
(22) Anmeldetag: 27.05.2010
(51) Int. Cl.: A61N 5/06

(54) **Warmwassergerät mit Leuchtmittel**

(30) Priorität: 27.05.2009 CN 200910033982
(71) Anmelder: BSH Bosch und Siemens Hausgeräte GmbH, 81739 München (DE)
(72) Erfinder: Schröder, Bernd, 210008, Nanjing Jiangsu (CN)

(57) **Zusammenfassung**

Die vorliegende Erfindung betrifft ein Warmwassergerät (100), umfassend: einen in einem Gehäuse (2) angeordneten Wasserspeicher (1) zum Aufnehmen bzw. Speichern von zu erwärmenden Wasser, einen Wassereinlauf (11) zum Einspeisen des Wasserspeichers (1), einen Wasserablauf (12) zum Entnehmen von Warmwasser aus dem Wasserspeicher (1), wobei ein am Gehäuse (2) angeordnetes Leuchtmittel (4) zum Ausstrahlen von verschiedenfarbigem Licht und eine Steuereinrichtung (3) zum Steuern des Leuchtmittels (4), die dergestalt eingerichtet ist, dass das Leuchtmittel (4) verschiedenfarbiges Licht gemäß einer bestimmten Farbabfolge ausstrahlt, vorgesehen sind.

## Beschreibung

### Hintergrund der Erfindung

Die vorliegende Erfindung betrifft ein Warmwassergerät bzw. einen Wassererhitzer gemäß dem Oberbegriff des Patentanspruchs 1.

### Hintergrund der Erfindung

Lichttherapien werden eingesetzt, um Krankheitssyndrome vorzubeugen, diese zu heilen oder zu lindern. Dies wird erreicht indem der menschliche Körper über einen spezifischen Zeitraum oder während einer spezifischen Tageszeit Licht ausgesetzt wird, das von einer natürlichen Lichtquelle oder einer künstlichen Lichtquelle mit einer spezifischen Wellenlänge ausgestrahlt wird. Es ist ein übliches angewendetes physisches Therapieverfahren, zum Beispiel ultraviolette Lichtstrahlung zum Heilen von Hautkrankheiten und infrarotes Licht zum Heilen von Knorpelgewebeschäden, Verschleiß und Arthritis einzusetzen. Ferner kann das Lichtausstrahlen mit verschiedenen bzw. unterschiedlichen Farben verwendet werden, um die menschliche Stimmung zu beeinflussen. Zum Beispiel werden Menschen durch Aussetzen gegenüber roter Lichtstrahlung belebt, bei welcher das zentrale Nervensystem stimuliert und der Blutkreislauf beschleunigt wird. Aussetzen gegenüber gelbem Licht lässt Leute sich fröhlich und lebhaft fühlen. Grünes Licht ist wirksam bei der Sedierung, und wirkt beruhigend. Blaues Licht hat ferner die Wirkung, die Stimmung behaglich zu machen und den Leuten bzw. Menschen zu helfen, sich zu entspannen.

Im Stand der Technik wird üblicherweise eine Lichttherapielampe verwendet, um die oben erwähnten Wirkungen zu erzielen. Gewöhnlich ist eine Lichttherapielampe in einem Badezimmer installiert, so dass Benutzer die Lichttherapie während, vor oder nach dem Bad unbekleidet durchführen können. Jedoch ist der Platz im Badezimmer bei einer gewöhnlichen Familie beschränkt. Die eingebaute Lichttherapielampe nimmt zusätzlichen Platz ein und macht den ohnehin beschränkten Platz noch enger.

Das chinesische Gebrauchsmuster CN98234740.5 offenbart einen elektrischen Wassererhitzer, in dem eine Leuchtstoffröhre eingebaut ist. Doch das Licht ist auf die Verwendung als Beleuchtung beschränkt und bietet nicht die Wirkung, Krankheitssyndrome zu heilen oder die Stimmung zu beeinflussen.

### Der Erfindung zugrundeliegende Aufgabe

Es ist eine Aufgabe der vorliegenden Erfindung, ein Warmwassergerät bereitzustellen, welches die Handhabung durch einen Benutzer verbessert.

### Erfindungsgemäße Lösung

Die Lösung der Erfindung zugrundeliegenden Aufgabe gelingt durch ein Warmwassergerät gemäß dem Oberbegriff des Patentanspruchs 1, das ein am Gehäuse angeordnetes Leuchtmittel zum Ausstrahlen von verschiedenfarbigem Licht und eine Steuereinrichtung zum Steuern des Leuchtmittels, die dergestalt eingerichtet ist, dass das Leuchtmittelverschiedenfarbiges Licht gemäß einer bestimmten Farbabfolge ausstrahlt. Folglich kann das Leuchtmittel zwei Funktionen bewirken, nämlich eine Beleuchtungsfunktion eines Raumes (beispielsweise eines Badezimmers) und weiter kann das Leuchtmittel zur Behandlung von Krankheitssyndromen, wie etwa Hauterkrankungen, oder zur Stimmungsbeeinflussung eines Benutzers eingesetzt werden.

### Bevorzugte Ausgestaltung der Erfindung

Es ist bevorzugt, dass das Leuchtmittel eine Beleuchtungseinheit aufweist, wobei die Steuereinrichtung dergestalt eingerichtet ist, dass die Beleuchtungseinheit ansteuerbar ist, um Licht mit einer ersten Farbe während eines ersten bestimmten Zeitintervalls und Licht mit einer zweiten Farbe während eines zweiten bestimmten Zeitintervalls auszustrahlen. Folglich kann ein flexibler Betrieb gewährleistet werden.

Vorteilhafterweise wiest das Leuchtmittel eine erste Beleuchtungseinheit und eine zweite Beleuchtungseinheit auf, wobei die Steuereinrichtung dergestalt eingerichtet ist, dass die erste Beleuchtungseinheit ansteuerbar ist, um Licht während des ersten bestimmten Zeitintervalls zu erzeugen, und dass die zweite Beleuchtungseinheit ansteuerbar ist, um Licht während des zweiten bestimmten Zeitintervalls auszustrahlen. Durch den Einsatz mehrerer Beleuchtungseinheiten kann die ausgestrahlte Farbabfolge beliebig variiert werden.

Es ist zweckmäßig, dass die erste Beleuchtungseinheit Licht mit einer ersten Farbe ausstrahlt, und die zweite Beleuchtungseinheit Licht mit einer zweiten Farbe ausstrahlt.

Es ist bevorzugt, dass das Leuchtmittel ferner eine erste Blende bzw. ein Abdeckungsteil mit einer ersten Farbe, welche die erste Beleuchtungseinheit abdeckt, sowie eine zweite Blende mit einer zweiten Farbe, welche die zweite Beleuchtungseinheit abdeckt, aufweist, wobei die erste Blende und die zweite Blende aus lichtdurchlässigen Materialien hergestellt sind. Die Verwendung von Blenden bzw. lichtdurchlässigen Abdeckungen kann eine kostengünstige Bereitstellung unterschiedlicher Farbabfolgen gewährleistet werden.

Gemäß einer Ausführungsform ist die erste Blende und die zweite Blende an dem Gehäuse angeordnet.

Vorteilhafterweise weist die Steuereinrichtung einen Speicher auf, der zum Speichern von spezifischen Farbabfolgedaten benutzbar ist. Somit kann das Gerät vorprogrammiert werden bzw. eine bevorzugte Farbabfolge kann im Speicher abgelegt werden.

Die Steuereinrichtung weist einen Mikrocontroller mit einem Zeitgebermodul auf. Somit kann eine genaue und vollautomatische Steuerung des Warmwassergeräts bereitgestellt werden.

Es ist bevorzugt, dass das Warmwassergerät weiter einen Modusschalter aufweist, der eingerichtet ist, um das Leuchtmittel ein- und/oder auszuschalten. Somit kann ein Benutzer durch Interaktion das Leuchtmittel steuern.

Gemäß einer anderen Ausführungsform ist ein weiterer Modusschalter vorgesehen, der eingerichtet ist, um aus einer Vielzahl von vorgegebenen Farbabfolgen, die durch das Leuchtmittel erzeugbar sind, auszuwählen. Somit kann ein Benutzer beispielsweise durch einfachen Knopfdruck die gewünschte Farbkombination oder Abfolge starten.

Weiter weist das Warmwassergerät einen Lautsprecher auf, der beispielsweise zum Abspielen von Musik einsetzbar ist.

Verglichen mit dem Stand der Technik schafft die vorliegende Erfindung unter anderem den Vorteil, dass der Wassererhitzer bzw. das Warmwassergerät mit dem Leuchtmittel Licht verschiedener Farben vorsieht und daher kann der Wassererhitzer Krankheitssyndromen vorbeugen, diese heilen oder lindern oder die Stimmung eines Benutzers nach Wunsch beeinflussen.

### Kurzbeschreibung der Zeichnungen

- Fig. 1: ist eine schematische Aufbauzeichnung einer ersten Ausführungsform des Wassererhitzers gemäß der vorliegenden Erfindung;
- Fig. 2: ist ein funktionelles Blockschaltbild eines Betriebsschaltkreises des in Fig. 1 gezeigten Wassererhitzers;
- Fig. 3: ist eine schematische Aufbauzeichnung einer zweiten Ausführungsform des Erhitzers gemäß der vorliegenden Erfindung;
- Fig. 4: ist ein funktionelles Blockschaltbild eines Betriebsschaltkreises des in Fig. 3 gezeigten Wassererhitzers; und
- Fig. 5: ist eine schematische Aufbauzeichnung einer dritten Ausführungsform des Erhitzers gemäß der vorliegenden Erfindung.

### Ausführliche Beschreibung

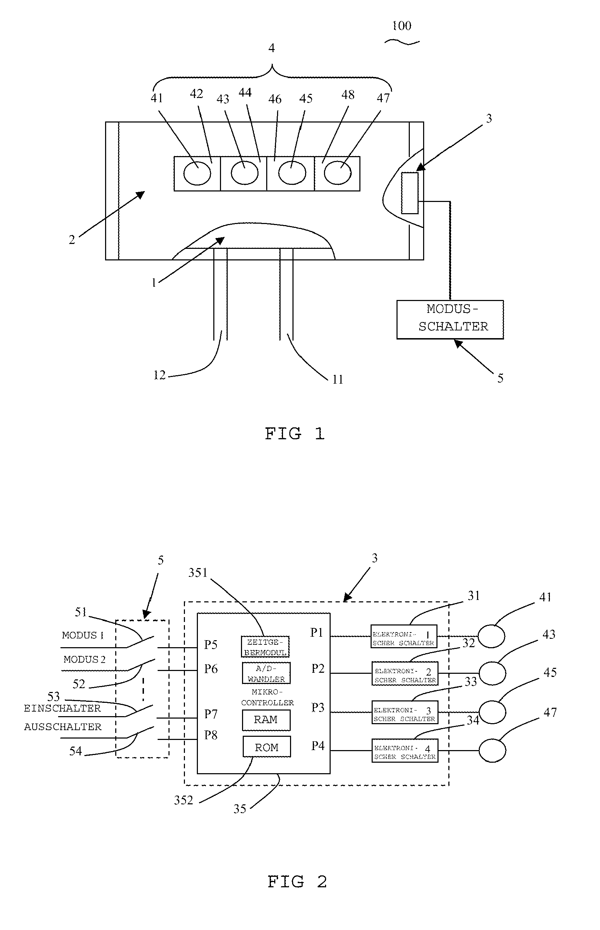

Eine erste Ausführungsform des Warmwassergeräts bzw. Wassererhitzers 100 gemäß der vorliegenden Erfindung ist in Fig. 1 und Fig. 2 gezeigt. In dieser Ausführungsform ist der Wassererhitzer 100 waagerecht angeordnet und umfasst einen zum Aufnehmen des zu erwärmenden Wassers benutzten Wasserspeicher 1, ein an der Außenfläche des Wasserspeichers 1 angeordnetes Gehäuse 2 und eine mit Schaummaterial gefüllte, zwischen dem Wasserspeicher 1 und dem Gehäuse 2 ausgebildete Isolationsschicht (in den Zeichnungen nicht gezeigt). Der Wasserspeicher 1 hat im Allgemeinen die Form eines Zylinders und ist gewöhnlich aus dunklem Metall, wie etwa Stahl oder Eisen hergestellt. Der Wasserspeicher 1 umfasst ein Einlassrohr bzw. einen Wassereinlauf 11, das zum Einlassen kalten Wassers in den Wasserspeicher benutzt wird, und ein Auslassrohr bzw. einen Wasserablauf 12, das zum Entnehmen von erwärmtem Wasser aus dem Wasserspeicher 1 benutzt wird. Der Wassererhitzer 100 umfasst auch eine Heizquelle (in den Zeichnungen nicht gezeigt), die zum Erwärmen von kaltem Wassers benutzt wird und in verschiedenen Formen ausgeführt sein kann. In einem Beispiel eines elektrischen Wassererhitzers ist ein Flansch an einem vertikalen Ende des Wasserspeichers 1 installiert, und ein elektrisches Heizrohr ist entlang dem Flansch installiert und erstreckt sich in das innerhalb des Wasserspeichers aufgenommene Wasser. Das Heizrohr kann einen Widerstandsdraht hoher Impedanz umfassen, und das positive und das negative Ende des Widerstandsdrahts sind an dem Flansch angeordnet und an eine Wechselstromquelle von 220 V oder 110 V angeschlossen. Fraglos gibt es auch Erhitzer, die andere Formen von Heizquellen benutzen, wie etwa Gas-Wassererhitzer, die Wasser innerhalb des Wasserspeichers über eine außerhalb des Wasserspeichers angeordnete Gasbrennkammer erwärmen.

Ein Leuchtmittel 4 bzw. Licht aussendende Vorrichtung 4 ist an dem Gehäuse 2 des Wassererhitzers 100 angeordnet. Das Leuchtmittel 4 ist am Gehäuse 2 dergestalt angeordnet, dass das durch die Licht aussendende Vorrichtung bzw. Leuchtmittel 4 ausgesendete Licht nicht durch das Gehäuse blockiert bzw. behindert wird. Zum Beispiel ist das Leuchtmittel 4 direkt am Gehäuse 2 angeordnet; oder innerhalb des Gehäuses 2, wo Poren und/oder Öffnungen, oder lichtdurchlässige Bereiche am Gehäuse 2 vorgesehen sind, um dem Licht zu erlauben, hindurchzutreten bzw. hindurch zu scheinen; oder das Leuchtmittel 4 ist teilweise innerhalb des Gehäuses 2 oder teilweise auf dem Gehäuse 2 angeordnet. In der vorliegenden Ausführungsform umfasst das Leuchtmittel 4 vier Beleuchtungseinheiten zum Aussenden von vier verschiedenen Farben, wie etwa eine erste Beleuchtungseinheit 41 zum Aussenden von rotem Licht, eine zweite Beleuchtungseinheit 43 zum Aussenden von gelbem Licht, eine dritte Beleuchtungseinheit 45 zum Aussenden von grünem Licht und eine vierte Beleuchtungseinheit 47 zum Aussenden von blauem Licht. Das Leuchtmittel 4 umfasst auch den ersten zweiten, dritten und vierten Kappenteil bzw. lichtdurchlässige Blende 42, 44, 46 bzw. 48, welche die vier Beleuchtungseinheiten 41, 43, 45, 47 abdecken. Davon sind die Beleuchtungseinheiten 41, 43, 45, 47 innerhalb des Gehäuses 2 angeordnet, und die Kappenteile bzw. lichtdurchlässige Blenden 42, 44, 46, 48 sind an dem Gehäuse 2 angeordnet. Die Beleuchtungseinheiten sind Hochspannungs- oder Niederspannungs-Entladungslampen, Halogenlampen, Glühlampen, Leuchtröhren, Fluoreszenzlampen, Halbleiter-Beleuchtungseinheiten oder beliebige andere verwendbare Beleuchtungseinheiten. Der Kappenteil ist aus lichtdurchlässigen Materialien, wie etwa Glas oder Kunststoff, hergestellt. In anderen Ausführungsformen sind die vier Beleuchtungseinheiten solche, die weißes Licht aussenden aussstrahlen, und der Kappenteil (Blende) ist aus lichtdurchlässigen Materialien verschiedener Farben hergestellt. Zum Beispiel ist der erste Kappenteil 42 aus rotem lichtdurchlässigem Glas, der zweite Kappenteil 44 aus gelbem lichtdurchlässigem Glas, der dritte Kappenteil 46 aus grünem lichtdurchlässigem Glas und der vierte Kappenteil 48 aus blauem lichtdurchlässigem Glas hergestellt. Solche Gestaltungen ermöglichen, dass die Licht aussendende Vorrichtung 4 Licht verschiedener Farben erzeugt. Außerdem ist auch anwendbar, verschiedene Farben mit derselben Beleuchtungseinheit auszusenden, wie etwa einer Leuchtdiode (LED) mit Licht aussendenden Dioden mit zwei Farben. Auch umfasst das Leuchtmittel 4 nur eine Beleuchtungseinheit und braucht keinen Kappenteil zu umfassen.

Mit Bezugnahme auf Fig. 2 sind ein Steuersystem bzw. eine Steuereinrichtung 3 und Beleuchtungseinheiten 41, 43, 45, 47 elektrisch verbunden. Das Steuersystem 3 umfasst vier elektronische Schalter 31, 32, 33, 34, die jeweils mit vier Beleuchtungseinheiten 41, 43, 45, 47 verbunden sind, sowie eine mit diesen elektronischen Schaltern verbundene programmierbare Steuereinheit zum Steuern des Ein-/Aus-Zustands der elektronischen Schalter. Davon sind die elektronischen Schalter 31, 32, 33, 34 Transistoren, Thyristoren, Relais oder Metall-Oxid-Halbleiter-Feldeffekttransistoren (MOSFETs) usw. Die programmierbare Steuereinheit ist ein Mikrocontroller oder eine integrierte Schaltung, wie etwa eine anwendungsspezifische integrierte Schaltung (ASIC) oder eine im Feld programmierbare Anordnung von Logikgattern(FPGA) usw. In der vorliegenden Ausführungsform ist die programmierbare Steuereinheit ein Mikrocontroller. Der Mikrocontroller umfasst eine Zentraleinheit (CPU), einen Festspeicher (ROM) 352, einen Arbeitsspeicher (RAM), ein Zeitgebermodul 351, einen A/D-Wandler und eine Vielzahl von Eingangs-/Ausgangsanschlüssen P1-P8 usw. Als bevorzugte Ausführung umfasst der Wassererhitzer 100 auch einen Modusschalter 5, der mit dem Steuersystem 3 verbunden ist, das zum Steuern des Ein- und Ausschaltens der Licht aussendenden Vorrichtung 4 benutzt wird. Weiter wählt der Modusschalter 5 auch verschiedene Lichtaussendungsmodi. In der vorliegenden Ausführungsform umfasst der Modusschalter 5 eine Vielzahl von Knöpfen. Es ist möglich andere Formen von Modusschaltern 5 einzusezen, wie etwa einen Drehschalter, der mehrere Drehabschnitte aufweist, oder einen einpoligen Umschalter mit mehreren Stellungen usw. Der Modusschalter 5 ist am Gehäuse 2 des Wassererhitzers angebaut oder an der Wand installiert. Der Modusschalter 5 ist auch elektrisch mit dem Steuersystem 3 über einen verbindenden Leitungsdraht verbunden; oder der Modusschalter 5 kann auch ein Fernsteuerschalter sein, der einen darin angeordneten Funksender aufweist, und das Steuersystem kann weiter einen Funkempfänger zum drahtlosen Steuern des Leuchtmittels 4 umfassen.

Das Folgende erläutert weiter den Steuermechanismus des Steuersystems 3 im Einzelnen. Wenn ein Benutzer das Leuchtmittel 4 benutzen möchte, drückt der Benutzer den EIN-Knopf 53, um die Licht aussendende Vorrichtung 4 einzuschalten. Danach wählt der Benutzer einen Lichtaussendungsmodus nach eigenen Wünschen. Wenn der Benutzer zum Beispiel den ersten Modusknopf 51 drückt, empfängt der Anschluss P5 am Mikrocontroller 35 ein High-Signal, dann wählt eine CPU einen entsprechenden Lichtaussendungsmodus (d.h. eine spezifische Abfolge bzw. Farbabfolge, in der jeweils Licht verschiedener Farben erzeugt wird) aus dem ROM 352 und steuert den Lichtaussendebetrieb der Licht aussendenden Vorrichtung 4. Zum Beispiel wird unter dem ersten Modus das rote Licht dauernd für zwei Minuten ausgestrahlt, anschließend wird dann das grüne Licht für fünf Minuten ausgesendet, dann wird das gelbe Licht für drei Minuten ausgestrahlt. Der Mikrocontroller 35 steuert den ersten elektronischen Schalter 31 so an, dass er die erste Beleuchtungseinheit 41 über den Anschluss P1 ansteuert. Gleichzeitig zählt das Zeitgebermodul 351 die Dauer von zwei Minuten hoch. Wenn die Zeitvorgabe von zwei Minuten abgelaufen ist, trennt der Mikrocontroller 35 am Anschluss P1 und steuert den dritten elektronischen Schalter 33 so an, dass er die dritte Beleuchtungseinheit 45 über den Anschluss P3 ansteuert. Zu dem Zeitpunkt wird das Zeitgebermodul 351 zurückgesetzt, um bis fünf Minuten zu zählen. Wenn die Zeitvorgabe von fünf Minuten abgelaufen ist, trennt der Mikrocontroller 35 am Anschluss P3 und steuert den zweiten elektronischen Schalter 32 so an, dass er die zweite Beleuchtungseinheit 43 über den Anschluss P2 ansteuert. Zur selben Zeit wird das Zeitgebermodul 351 zurückgesetzt, um bis drei Minuten zu zählen. Ebenso wählt der Benutzer über einen Druck des zweiten Modusknopfes 52 eine andere Abfolge, um jeweils eine entsprechende Lichtaussendung bzw. Lichtausstrahlung von Farben auszulösen. Wenn der Benutzer das Leuchtmittel 4 nicht verwenden möchte, schaltet der Benutzer das Leuchtmittel 4 aus, indem er den AUS-Knopf 54 bedient.

In anderen Ausführungsformen wird die Beleuchtungseinheit angesteuert, um dauernd Licht oder flackerndes auszustrahlen. Zum Beispiel gibt der Mikrocontroller 35 über den Anschluss P1 ein Rechteckwellensignal aus, das eine spezifische Frequenz aufweist, um die erste Beleuchtungseinheit 41 so anzusteuern, dass sie mit der vorgegebenen Frequenz flackerndes Licht ausstrahlt. Außerdem kann der Modusschalter 5 mehr Modusknöpfe umfassen. Auch kann der Lichtausstrahlmodus Licht über nur eine Beleuchtungseinheit aussenden. Wenn sich ein Benutzer zum Beispiel entspannen oder seine Stimmung beruhigen möchte, kann der Benutzer einen entsprechenden Modusknopf betätigen, um den Mikrocontroller 35 zu befähigen, die dritte Beleuchtungseinheit 45 anzusteuern, grünes Licht abzugeben. In diesem Modus strahlt die dritte Beleuchtungseinheit 45 Licht über einen längeren vorgegebenen Zeitraum aus oder strahlt dauernd bzw. kontinuierlich Licht aus, bis der Benutzer die dritte Beleuchtungseinheit 45 von Hand ausschaltet. Oder wenn ein Benutzer zu einer fröhlichen oder angeregten Stimmung wechseln möchte, steuert der Benutzer die erste Beleuchtungseinheit 41 über den entsprechenden Modusknopf an, rotes Dauerlicht oder rotes Flackerlicht auszustrahlen oder zuerst über einen spezifischen Zeitraum rotes Dauerlicht und dann über einen weiteren spezifischen Zeitraum rotes Flackerlicht auszustrahlen. Außerdem ist es in einer weiteren Ausführungsform nicht erforderlich, Modusschalter anzuwenden. Mit anderen Worten, wenn der Wassererhitzer eingeschaltet wird, wird die Licht aussendende Vorrichtung gleichzeitig eingeschaltet und strahlt Licht gemäß einen vorgegebenen Lichtausstrahlmodus aus.

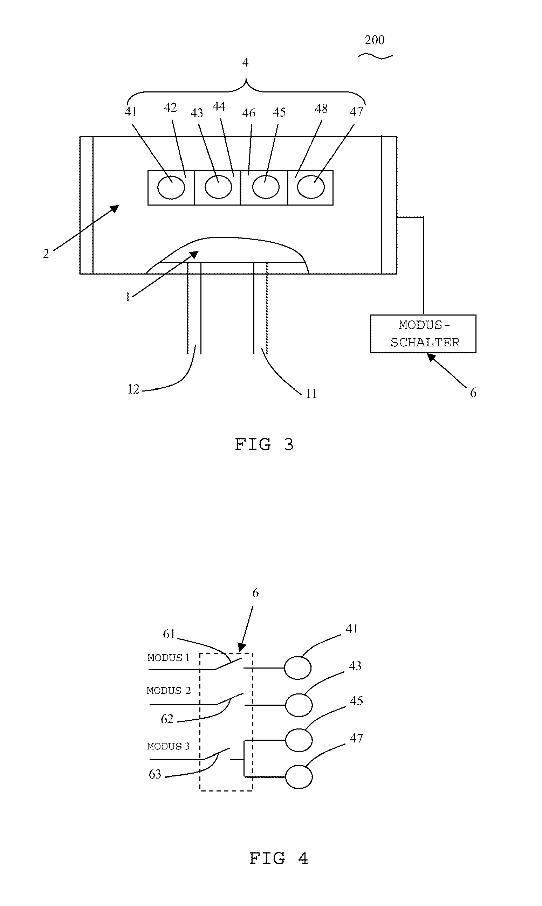

Fig. 3 und Fig. 4 offenbaren eine zweite Ausführungsform des Wassererhitzers gemäß der vorliegenden Erfindung. Im Vergleich zur ersten Ausführungsform liegt der Hauptunterschied darin, dass der Wassererhitzer 200 kein Steuersystem aufweist. Stattdessen steuert ein Modusschalter 6 direkt ein Leuchtmittel 4. Mit Bezugnahme auf Fig. 4 löst ein erster Modusknopf 61 direkt die erste Beleuchtungseinheit 41 aus, ein zweiter Modusknopf 62 löst direkt die zweite Beleuchtungseinheit 42 aus, ein dritter Modusknopf 63 löst sowohl die dritte als auch die vierte Beleuchtungseinheit 45, 47 aus. Der Wassererhitzer 200 der Ausführungsform ist besser für Benutzer geeignet, die Licht spezifischer Farbe benutzen müssen, um spezifische Krankheitssyndrome zu heilen.

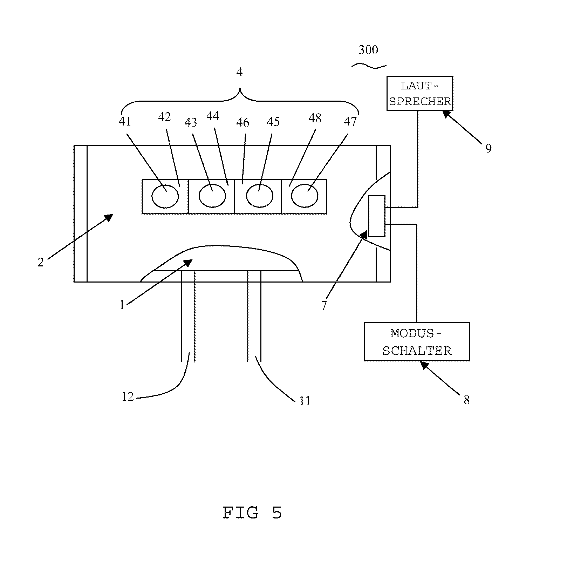

Fig. 5 offenbart eine dritte Ausführungsform des Wassererhitzers gemäß der vorliegenden Erfindung. In dieser Ausführungsform umfasst ein Wassererhitzer 300 auch einen Lautsprecher 9. Der Lautsprecher 9 ist an einer beliebigen Stelle am Wassererhitzergehäuse 2 angeordnet oder kann getrennt an der Wand angeordnet sein, und er ist mit dem Steuersystem 7 über einen verbindenden Leitungsdraht oder eine drahtlose Verbindung verbunden. Der Modusschalter 8 wird auch zur Auswahl eines abzuspielenden Klangs benutzt. Wenn ein Benutzer zum Beispiel über den Modusknopf den Entspannungsmodus wählt, steuert das Steuersystem 7 das Leuchtmittel 4 gemäß dem entsprechenden Lichtausstrahlmodus an, einfarbiges Licht oder Licht verschiedener Farben auszustrahlen, und gleichzeitig steuert das Steuersystem 7 auch den Lautsprecher 9 an, gemäß einem entsprechenden Audiomodus einen Klang von Vogelgezwitscher oder einem fließenden Bach abzuspielen. So fühlt sich der Benutzer, als wäre der Benutzer in der Natur, was nützlich ist beim Erzeugen einer behaglichen Umgebung bei der Therapie oder bei der Beeinflussung der Stimmung. Folglich stellt der Wassererhitzer mit Leuchtmittel Licht verschiedener Farben bereit, und kann daher Krankheitssyndromen vorbeugen, diese heilen oder lindern oder die Stimmung eines Benutzers nach Wunsch beeinflussen.

## Patentansprüche

1. Warmwassergerät (100), umfassend:
- einen in einem Gehäuse (2) angeordneten Wasserspeicher (1) zum Aufnehmen bzw. Speichern von zu erwärmenden Wasser;
- einen Wassereinlauf (11) zum Einspeisen des Wasserspeichers (1);
- einen Wasserablauf (12) zum Entnehmen von Warmwasser aus dem Wasserspeicher (1);
**gekennzeichnet durch**
- ein am Gehäuse (2) angeordnetes Leuchtmittel (4) zum Ausstrahlen von verschiedenfarbigem Licht; und
- eine Steuereinrichtung (3) zum Steuern des Leuchtmittels (4), die dergestalt eingerichtet ist, dass das Leuchtmittel (4) verschiedenfarbiges Licht gemäß einer bestimmten Farbabfolge ausstrahlt.

2. Warmwassergerät (100) nach Anspruch 1, **dadurch gekennzeichnet, dass** das Leuchtmittel (4) mindestens eine Beleuchtungseinheit (41, 43, 45, 47) aufweist, wobei die Steuereinrichtung (3) dergestalt eingerichtet ist, dass die Beleuchtungseinheit ansteuerbar ist, um Licht mit einer ersten Farbe während eines ersten bestimmten Zeitintervalls und Licht mit einer zweiten Farbe während eines zweiten bestimmten Zeitintervalls auszustrahlen.

3. Warmwassergerät (100) nach Anspruch 1, **dadurch gekennzeichnet, dass** Leuchtmittel (4) eine erste Beleuchtungseinheit (41, 43, 45, 47) und eine zweite Beleuchtungseinheit (41, 43, 45, 47) aufweist, wobei die Steuereinrichtung (3) dergestalt eingerichtet ist, dass die erste Beleuchtungseinheit (41, 43, 45, 47) ansteuerbar ist, um Licht während des ersten bestimmten Zeitintervalls zu erzeugen, und dass die zweite Beleuchtungseinheit (41, 43, 45, 47) ansteuerbar ist, um Licht während des zweiten bestimmten Zeitintervalls auszustrahlen.

4. Warmwassergerät (100) nach Anspruch 3, **dadurch gekennzeichnet, dass** die erste Beleuchtungseinheit (41, 43, 45, 47) Licht mit einer ersten Farbe ausstrahlt, und die zweite Beleuchtungseinheit (41, 43, 45, 47) Licht mit einer zweiten Farbe ausstrahlt.

5. Warmwassergerät (100) nach einem der vorhergehenden Ansprüche, **dadurch gekennzeichnet, dass** das Leuchtmittel (4) ferner mindestens eine erste Blende bzw. ein Abdeckungsteil (42, 44, 46, 48) mit einer ersten Farbe, welche die erste Beleuchtungseinheit abdeckt, sowie eine zweite Blende mit einer zweiten Farbe, welche die zweite Beleuchtungseinheit abdeckt, aufweist, wobei die erste Blende und die zweite Blende aus lichtdurchlässigen Materialien hergestellt sind.

6. Warmwassergerät (100) nach Anspruch 5, **dadurch gekennzeichnet, dass** die erste Blende (42, 44, 46, 48) und die zweite Blende (42, 44, 46, 48) an dem Gehäuse (2) angeordnet sind.

7. Warmwassergerät (100) nach einem der vorhergehenden Ansprüche, **dadurch gekennzeichnet, dass** die Steuereinrichtung (3) einen Speicher aufweist, der zum Speichern von spezifischen Farbabfolgedaten benutzbar ist.

8. Warmwassergerät (100) nach einem der vorhergehenden Ansprüche, **dadurch gekennzeichnet, dass** die Steuereinrichtung (3) einen Mikrocontroller mit einem Zeitgebermodul aufweist.

9. Warmwassergerät (100) nach einem der vorhergehenden Ansprüche, **dadurch gekennzeichnet, dass** das Warmwassergerät weiter einen Modusschalter (5) aufweist, der eingerichtet ist, um das Leuchtmittel ein- und/oder auszuschalten.

10. Warmwassergerät (100) nach einem der vorhergehenden Ansprüche, **dadurch gekennzeichnet, dass** ein weiterer Modusschalter (5) vorgesehen ist, der eingerichtet ist, um aus einer Vielzahl von vorgegebenen Farbabfolgen, die durch das Leuchtmittel (4) erzeugbar sind, auszuwählen.

11. Warmwassergerät (100) nach einem der vorhergehenden Ansprüche, **dadurch gekennzeichnet, dass** Warmwassergerät (100) einen Lautsprecher (9) umfasst.
